# EUROPÄISCHE PATENTSCHRIFT

(11) **EP 0 382 114 B1**
(45) Veröffentlichungstag und Bekanntmachung des Hinweises auf die Patenterteilung: **23.10.1996**
(21) Anmeldenummer: 90102069.3
(22) Anmeldetag: 02.02.1990
(51) Int. Cl.: C07F 9/32, C07F 9/30

(54) **Phosphinesterhaltige N-Acyl-2-aminosäureamide, Verfahren zu ihrer Herstellung und N-Acyl-2-aminosäurenitrile als Vorprodukte**
N-acyl-2-amino acid amides containing phosphinic-acid esters, process for their preparation and N-acyl-2-amino acid nitriles as starting products
Amides d'acides amino-2 carboxyliques n-acylés contenant un groupe ester phoshinique, procédé pour les préparer et nitriles d'acides amino-2 carboxyliques comme produits de départ

(30) Priorität: 06.02.1989 DE 3903446; 20.05.1989 DE 3916551
(43) Veröffentlichungstag der Anmeldung: 16.08.1990
(73) Patentinhaber: Hoechst Schering AgrEvo GmbH, 13342 Berlin (DE)
(72) Erfinder: Willms, Lothar, Dr., D-5416 Hillscheid (DE)

(56) Entgegenhaltungen:
- EP-A- 0 011 245
- BULLETIN OF THE CHEMICAL SOCIETY OF JAPAN, Band 61, Nr. 10, Oktober 1988, Seiten 3699-3704, The Chemical Society of Japan; I.A. NATCHEV: "Organophosphorus analogues and derivatives of the natural L-amino carboxylic acids and peptides. I. Enzymatic synthesis of D-,DL-, and L-phosphinothricin and their cyclic analogues"

## Beschreibung

Die vorliegende Erfindung betrifft phosphorhaltige N-Acyl-2-aminosäureamide der allgemeinen Formel (I) 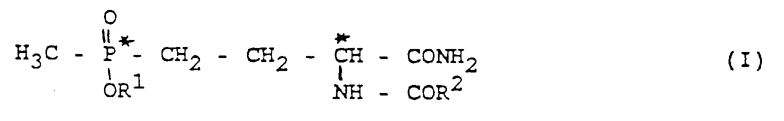 worin
- R¹: C₁-C₁₄-Alkyl, welches verzweigt oder unverzweigt ist und unsubstituiert oder ein- oder mehrfach durch Halogen oder C₁-C₆-Alkoxy substituiert ist, Benzyl oder Phenyl, wobei Benzyl oder Phenyl im Phenylkern unsubstituiert oder ein- oder mehrfach durch C₁-C₄-Alkyl, C₁-C₄-Alkoxy, Halogen, Nitro oder Trifluormethyl substituiert sind, oder C₃-C₁₀-Cycloalkyl und
- R²: Wasserstoff, C₁-C₁₄-Alkyl, welches unsubstituiert ist oder ein- oder mehrfach durch Halogen oder C₁-C₄-Alkoxy substituiert ist, oder einen Rest der Formel -(CH₂)ₙ-Phenyl, der im Phenylkern unsubstituiert oder ein- bis dreifach durch C₁-C₄-Alkyl, C₁-C₄-Alkoxy, Halogen, Nitro oder Trifluormethyl substituiert ist und in dem n für 0, 1, 2 oder 3 steht,
bedeuten.

Verbindungen der Formel (I), worin R¹ = Ethyl oder Methyl und R² = Methyl bzw. Trifluormethyl bedeuten, und deren Herstellung aus N-acyliertem 3-Amino-pyrrolidon-(2) und Methylphosphinsäureester sind bereits aus Bull. Chem. Soc. Jpn. **61**, 3699-3704 (1988) bekannt. Die Herstellung von Phosphinothricin durch Hydrolyse jeweils eines der Produkte mit Salzsäure, Ammoniak oder durch drei aufeinanderfolgende enzymatische Hydrolysen mit Phosphodiesterase, Acylase I und Glutaminase ist dort ebenfalls beschrieben.

Von besonderem Interesse sind Verbindungen der Formel (I), worin
- R¹: unverzweigtes oder verzweigtes C₁-C₁₀-Alkyl oder C₁-C₁₀-Alkyl, das durch Halogen, wie Fluor oder Chlor, ein- bis dreifach substituiert ist, oder C₅-C₆-Cycloalkyl und
- R²: Wasserstoff, unverzweigtes oder verzweigtes . C₁-C₁₄-Alkyl, das unsubstituiert oder durch ein bis drei Reste aus der Gruppe Halogen oder C₁-C₄-Alkoxy substituiert ist, oder ein Rest der Formel -(CH₂)ₙ-Phenyl, wobei der Phenylkern unsubstituiert oder ein- bis dreifach durch Halogen substituiert ist und n für 0, 1 oder 2 steht,
bedeuten.

Bevorzugt sind Verbindungen der Formel (I), worin
- R¹: unverzweigtes C₁-C₈-Alkyl oder Cyclohexyl und
- R²: C₁-C₄-Alkyl oder Benzyl
bedeuten.

Als Bedeutung für R¹ kommen beispielsweise die Reste Methyl, Ethyl, n-Propyl, i-Propyl, n-, i-, t- und 2-Butyl, n-, i-, t-, 2-, 3- und neo-Pentyl, n-, i- und 2-Hexyl, Heptyl, Octyl, Nonyl, Decyl, Undecyl, Dodecyl, Tridecyl, Tetradecyl, 2-Chlorethyl, 2,2-Dichlorethyl, 1,1,2,2-Tetrafluorethyl, 2-Methoxyethyl, 1-Ethoxyethyl, 2,2-Dimethoxy-ethyl, 3-Methoxypropyl, Benzyl, Phenyl, o-und p-Tolyl, 2-Methoxyphenyl, 4-Nitrophenyl, 2- oder 3-Chlor-phenyl, 4-Trifluormethyl-phenyl, 4-Chlorbenzyl, Cyclopropyl, Cyclobutyl, Cyclopentyl, Cyclohexyl, 4,4-Dimethylcyclohexyl, Cycloheptyl, Cyclooctyl in Frage.

Für R² kommen beispielsweise die Bedeutungen Wasserstoff, Reste wie für R¹ aufgeführt, 1- und 2-Phenylethyl, 1-Phenyl-propyl, 3-Phenylpropyl und 1-(2-Chlorphenyl)-ethyl in Frage. Halogen bedeutet jeweils Fluor, Chlor und/oder Brom, vorzugsweise Fluor und/oder Chlor.

Gegenstand der Erfindung ist auch ein Verfahren zur Herstellung der erfindungsgemäßen Verbindungen der Formel (I), dadurch gekennzeichnet, daß man N-Acyl-2-aminosäurenitrile der Formel (II), 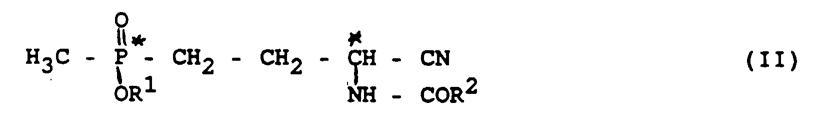 worin
R¹ und R² die für Formel (I) angegebenen Bedeutungen haben, selektiv an der Nitril-Gruppe zum entsprechenden Carbonsäureamid der allgemeinen Formel (I) sauer hydrolysiert.

In den Verbindungen der Formeln (I) und (II) liegen zwei Chiralitätszentren vor, so daß nach dem erfindungsgemäßen Verfahren entweder die Gemische von Diastereomeren, Enantiomeren oder einzelne Diastereomere bzw. Enantiomere der Formel (II) eingesetzt werden können. Die Hydrolyse an der Nitrilgruppe verläuft praktisch unter Erhalt der Konfiguration an den beiden genannten Chiralitätszentren. Wird beispielsweise ein α-D-Isomer der Formel (II) eingesetzt, das bezüglich des Chiralitätszentrums in α-Stellung zur Nitrilgruppe optisch rein ist und nur bezüglich deß Chiralitätszentrums an der Phosphinsäureestergruppe als Stereoisomerengemisch vorliegt, so erhält man nach der erfindungsgemäßen Hydrolyse der Nitrilfunktion das α-D-N-Acylaminosäureamid der allgemeinen Formel (I) unter Erhalt der Konfiguration (optischen Aktivität) bezüglich der α-Stellung zur Nitrilgruppe.

Gegenstand der Erfindung sind auch Verbindungen der Formel (II), die als Zwischenprodukte zur Herstellung der erfindungsgemäßen Verbindungen der Formel (I) verwendet werden können und in denen R¹ und R² die für Formel (I) genannten Bedeutungen haben. Vorzugsweise haben R¹ und R² in Formel (II) die für Formel (I) bevorzugten Bedeutungen.

Die für die Herstellung der erfindungsgemäßen Verbindungen der allgemeinen Formel (I) benötigten Ausgangsstoffe der Formel (II) lassen sich beispielsweise durch Acylierung von α-Aminosäurenitrilen der allgemeinen Formel (III) 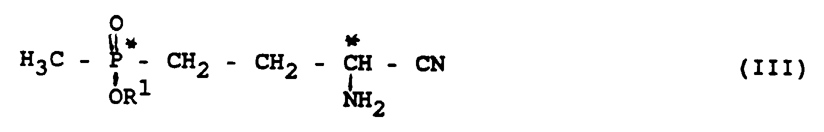 mit Säurechloriden der Formel R²-COCl beziehungsweise Säureanhydriden der Formel (R²CO)₂O nach an sich üblichen Verfahren herstellen (vergleiche Organikum, VEB Deutscher Verlag der Wissenschaften, Berlin 1976, 512 ff). Die Aminosäurenitrile der Formel (III) sind aus EP-A 194 521 (US-A-4,692,541) und EP-A 011 245 (US-A-4,521,348) bekannt oder lassen sich beispielsweise nach den in dieser Literatur bekannten Verfahren herstellen.

Das erfindungsgemäße Verfahren wird so durchgeführt, daß man Verbindungen der Formel (II) unter geeigneten Reaktionsbedingungen selektiv an der Nitrilfunktion zum entsprechenden Carbonamid der Formel (I) hydrolysiert, wobei die N-Acyl- bzw. Phosphinester-Gruppe nicht oder nicht wesentlich angegriffen oder hydrolysiert werden. Das Verfahren wird beispielsweise in Ameisensäure, gegebenenfalls in Gegenwart von Lewis-Säuren wie Halogenwasserstoffen, z.B. Chlorwasserstoff oder Bromwasserstoff, gegebenenfalls in Anwesenheit eines inerten organischen Lösungsmittels, wie z.B. Dichlormethan, Toluol oder Chlorbenzol, durchgeführt. Analoge Reaktionsbedingungen sind aus Liebigs Annalen der Chemie 713, 212 (1968); ibid. 749, 198 (1971); J. Chem. Soc. [C], 1970, 1230 bekannt. Die Ameisensäure kann in 100 %iger Form oder in geringerer Konzentration, d.h. mit Wasser verdünnt, eingesetzt werden. Die Ameisensäure wird in Mengen von 90 bis 600 Mol-%, bevorzugt in äquimolaren Mengen oder einem 2- bis 3fachen Überschuß, bezogen auf die Verbindung der Formel (II) eingesetzt.

Das erfindungsgemäße Verfahren wird bevorzugt bei Reaktionstemperaturen von 0 - 260°C, insbesondere bei weniger als 250°C unter Druck unter Verwendung von Ameisensäure ohne Lewis-Katalysatoren oder bei 0 - 60°C unter Verwendung von Ameisensäure in Gegenwart von Lewis-Katalysatoren durchgeführt. Die Lewis-Säuren werden in der Regel in katalytischen Mengen, beispielsweise 0,1 bis 2 Mol-%, vorzugsweise etwa 0,5 Mol-%, oder aber im Überschuß, beispielsweise 150 bis 600 Mol-% eingesetzt.

Die Reaktionsdauer kann je nach Reaktionsbedingungen sehr verschieden sein, beträgt jedoch in der Regel 0,1 bis 48 Stunden, vorzugsweise 0,5 bis 6 Stunden.

Das erfindungsgemäße Herstellungsverfahren für Verbindungen der Formel (I) ist überraschend selektiv. Die N-Acylaminosäurenitrile der allgemeinen Formel (II) werden zu den N-Acylaminosäureamiden der allgemeinen Formel (I) selektiv gespalten, ohne daß beispielsweise die säurelabile Phosphinestergruppierung oder die ebenfalls unter sauren Bedingungen spaltbare N-Acylgruppierung wesentlich angegriffen werden. Aus der DE-A 2 717 440 (GB-A-1,587,292) oder der EP-A 011 245 sind Verfahren bekannt, wonach beispielsweise der nach der Strecker-Synthese hergestellte 3-Amino-3-cyano-propyl(methyl)phosphinsäureethylester mit Salzsäure vollständig zu der entsprechenden 3-Amino-3-carboxy-propyl(methyl)phosphinsäure gespalten wird. Aus Liebigs Ann. Chem. 749, 198 (1971) ist bekannt, daß (2-Cyano-ethyl)phosphonsäuredimethylester in Ameisensäure/Chlorwasserstoff zu (2-Amidocarbonyl-ethyl)phosphonsäure gespalten wird und dabei eine partielle Verseifung zu (2-Amidocarbonyl-ethyl)phosphonsäuremono- bzw. Dimethylester offensichtlich nicht stattfindet. Im Vergleich dazu gestattet das erfindungsgemäße Verfahren die Herstellung phosphinsäureesterhaltiger Carboxamide der allgemeinen Formel (I) in nahezu quantitativen Ausbeuten, d.h. praktisch ohne Verseifung an der Phosphinestergruppe.

Die N-Acyl-α-aminosäureamide der allgemeinen Formel (I) stellen wertvolle Vorprodukte dar, die sich enzymatisch zu L-Phosphinothricin mit hohen Enantiomerenüberschüssen spalten lassen (vgl. Deutsche Patentanmeldung P 3903446.1, HOE 89/F 042). L-Phosphinothricin besitzt bakterizide (Helv. Chim. Acta. 55, 224 (1972)), fungizide (Sci. Rep. Meiji Seika Kaisha 13, 34 (1973)) und ausgezeichnete herbizide Eigenschaften (DE-A 2 717 440, EP-A 54897).

Die nachfolgenden Beispiele sollen die Erfindung näher erläutern, ohne sie auf die konkreten Beispiele zu beschränken.

### Beispiel 1:

### D,L-(3-Phenylacetamido-3-aminocarbonyl-propyl)methylphosphinsäurecyclohexylester

a) D,L-(3-Acetoxy-3-cyano-propyl)methylphosphinsäurecyclohexylester (Herstellung analog EP-11245);
   zu 130 g (0,8 Mol) Methanphosphonigsäuremonocyclohexylester werden bei 120°C unter Stickstoff-Atmosphäre innerhalb von 1 Stunde 50 g (0,4 Mol) Acroleincyanhydrinacetat getropft, das 4 g t-Butylperoctoat enthält. Nach beendetem Zutropfen wird 15 Minuten bei 120°C nachgerührt und anschließend unter reduziertem Druck fraktioniert destilliert. Man erhält 106 g (92 % d. Th.) D,L-(3-Acetoxy-3-cyanopropyl)methylphosphinsäurecyclohexylester vom Siedepunkt 178 bis 180°C bei 0,02 mbar.
b) D,L-(3-Phenylacetamido-3-cyano-propyl)methylphosphinsäurecyclohexylester;
   28,7 g (0,1 Mol) D,L-(3-Acetoxy-3-cyano-propyl)methylphosphinsäurecyclohexylester werden bei 20°C in 1 Stunde zu 29,6 ml konzentriertem Ammoniak getropft. Anschließend wird das Reaktionsgemisch mit Methylenchlorid extrahiert, der Extrakt über Natriumsulfat getrocknet und mit 10,2 g (0,1 Mol) Triethylamin versetzt. Bei 0°C werden 15,4 g (0,1 Mol) Phenylessigsäurechlorid zugetropft. Nach 18 Stunden Rühren bei Raumtemperatur wird mit 50 ml Wasser versetzt, mit 0,5 N Salzsäure pH 5 eingestellt und mit Methylenchlorid extrahiert. Das nach dem Eindampfen des Methylenchlorid-Extrakts verbleibende Öl wird durch Chromatographie an Kieselgel (Laufmittel Methylenchlorid) gereinigt. Man erhält 27,5 g (76 % d. Th.) an D,L-(3-Acetoxy-3-cyano-propyl)methylphosphinsäurecyclohexylester;
   ¹H-NMR (CDCl₃): δ=9,45 (NH, m, 1H); 7,3 (C₆H₅, s, 5H); 4,95 (CH, m, 1H); 4,36 (CH, m, 1H); 3,6 (CH₂, s, 2H); 1,2 - 2,2 (CH₂CH₂, PCH₃, C₆H₁₀, m, 17H).
c) D,L-(3-Phenylacetamido-3-aminocarbonyl-propyl)methylphosphinsäurecyclohexylester;
   6 g (0,01 Mol) D,L-(3-Phenylacetamido-3-cyano-propyl)methylphosphinsäurecyclohexylester werden in 40 ml Ameisensäure gelöst. Danach wird bei Raumtemperatur HCl-Gas eingeieitet. Nach 3 Stunden wird das Reaktionsgemisch eingeengt, der Rückstand in Methylenchlorid und Wasser gelöst und mit Natriumhydrogencarbonat ein pH-Wert von 5 eingestellt. Nach Extrahieren mit Methylenchlorid werden die Methylenchlorid-Extrakte über Natriumsulfat getrocknet und einrotiert. Das verbleibende Rohprodukt wird durch Chromatographie an Kieselgel (Laufmittel Methylenchlorid/Methanol 9:1) gereinigt. Man erhält 5,90 g (94,1 % d. Th.) eines hellgelben Öls;
   ¹H-NMR (CDCl₃): δ=7,2 (C₆H₅, s, 5H); 7,2 (CONH₂, d, 2H); 5,6 (NH, s, 1H); 4,2 - 4,8 (2 x CH, m, 2H); 3,6 (CH₂, s, 2H); 1,2 - 2,3 (CH₂CH₂, PCH₃, C₆H₁₀, m, 17H).

Die Substanz kristallisiert nach einigen Wochen zu einem feinkristallinen farblosen Pulver mit Schmelzpunkt von 128 bis 130°C.

### Beispiele 2b) bis 50b)

Analog zu dem in Beispiel 1 b) beschriebenen N-Acyl-α-aminosäurenitril lassen sich die in der folgenden Tabelle 1 genannten Verbindungen der allgemeinen Formel (II) herstellen.

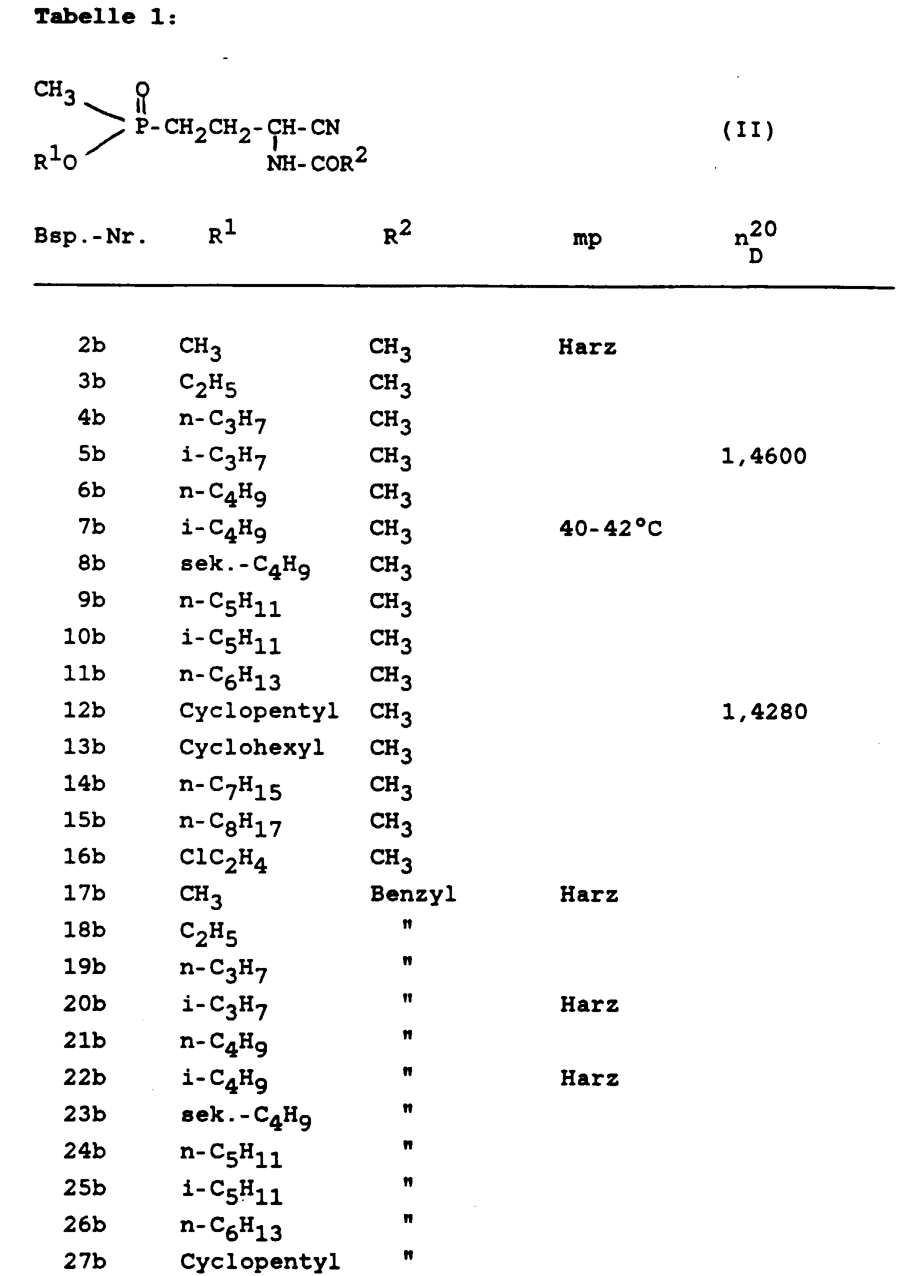

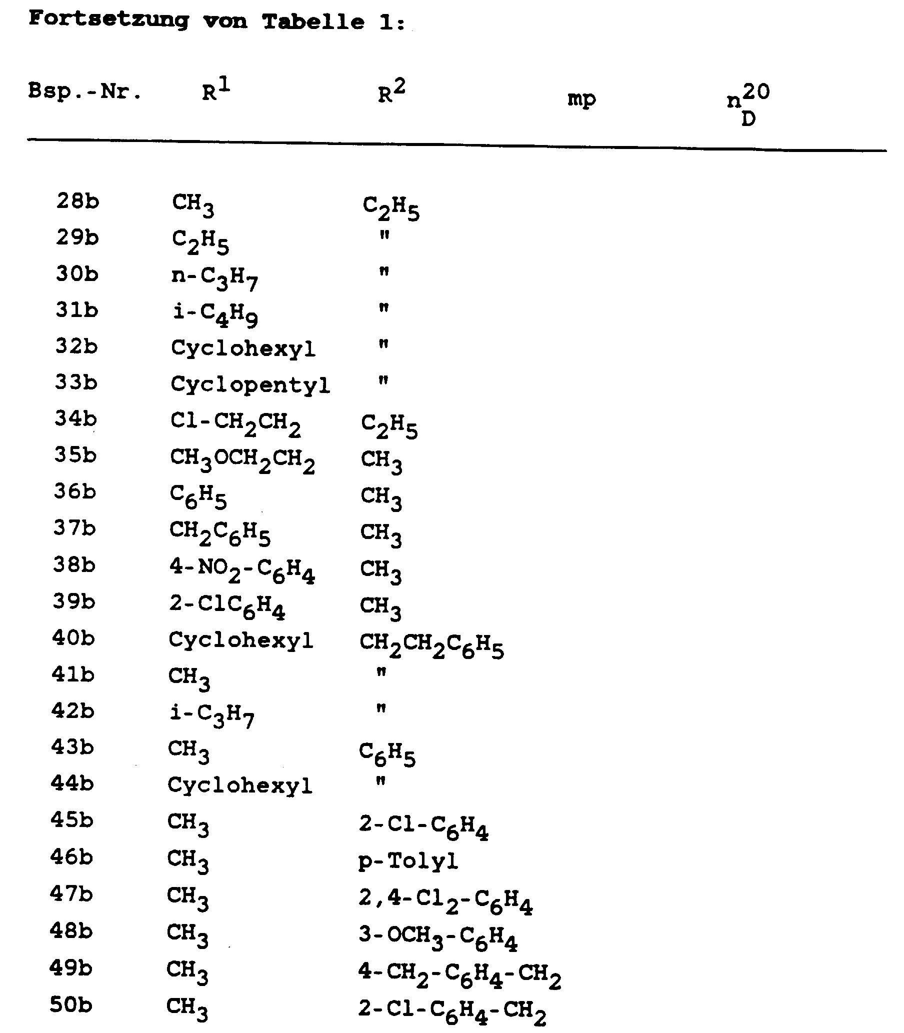

### Beispiele 2c) bis 50c)

Analog dem in Beispiel 1 c) beschriebenen N-Acyl-α-aminosäureamid lassen sich beispielsweise die in Tabelle 2 aufgeführten Verbindungen der allgemeinen Formel (I) herstellen.

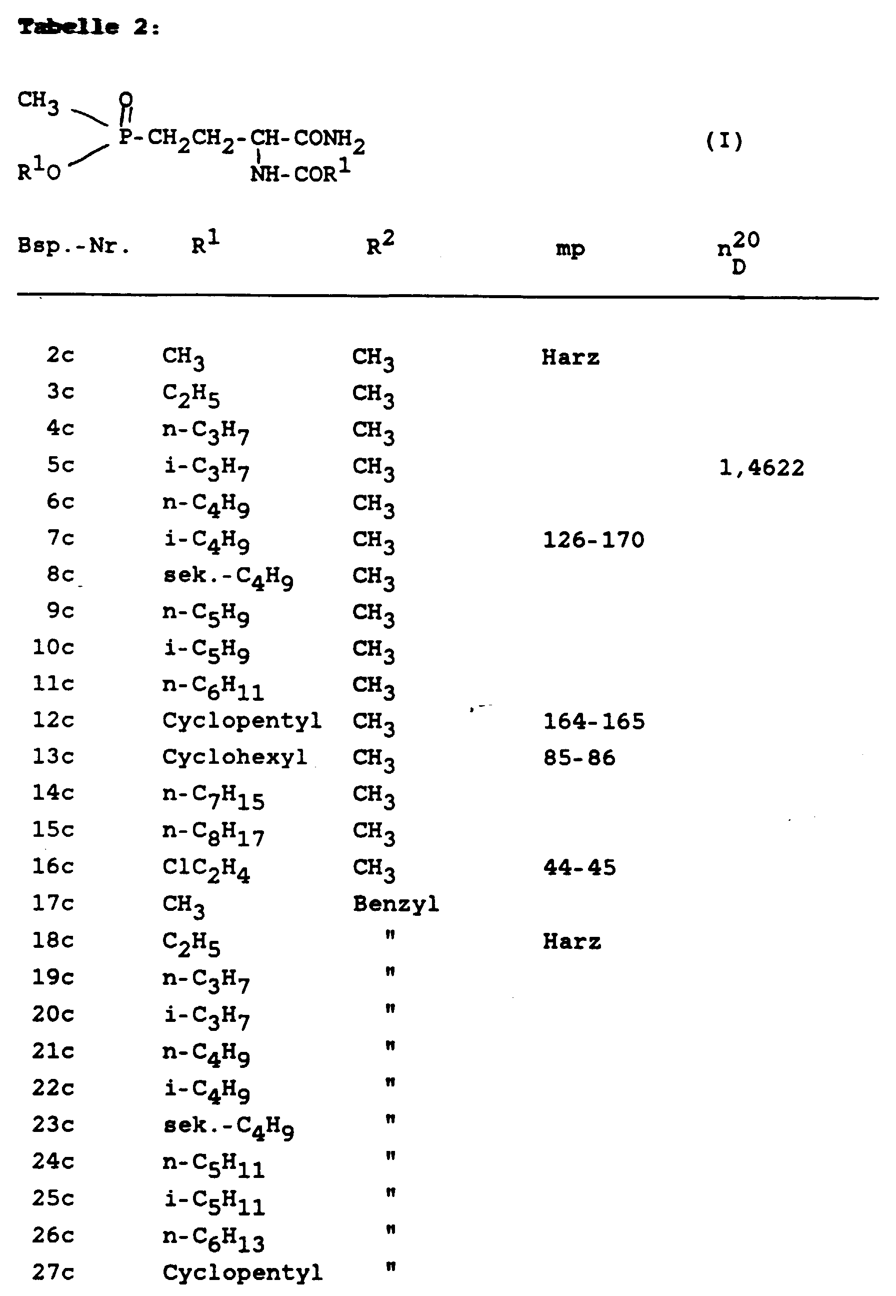

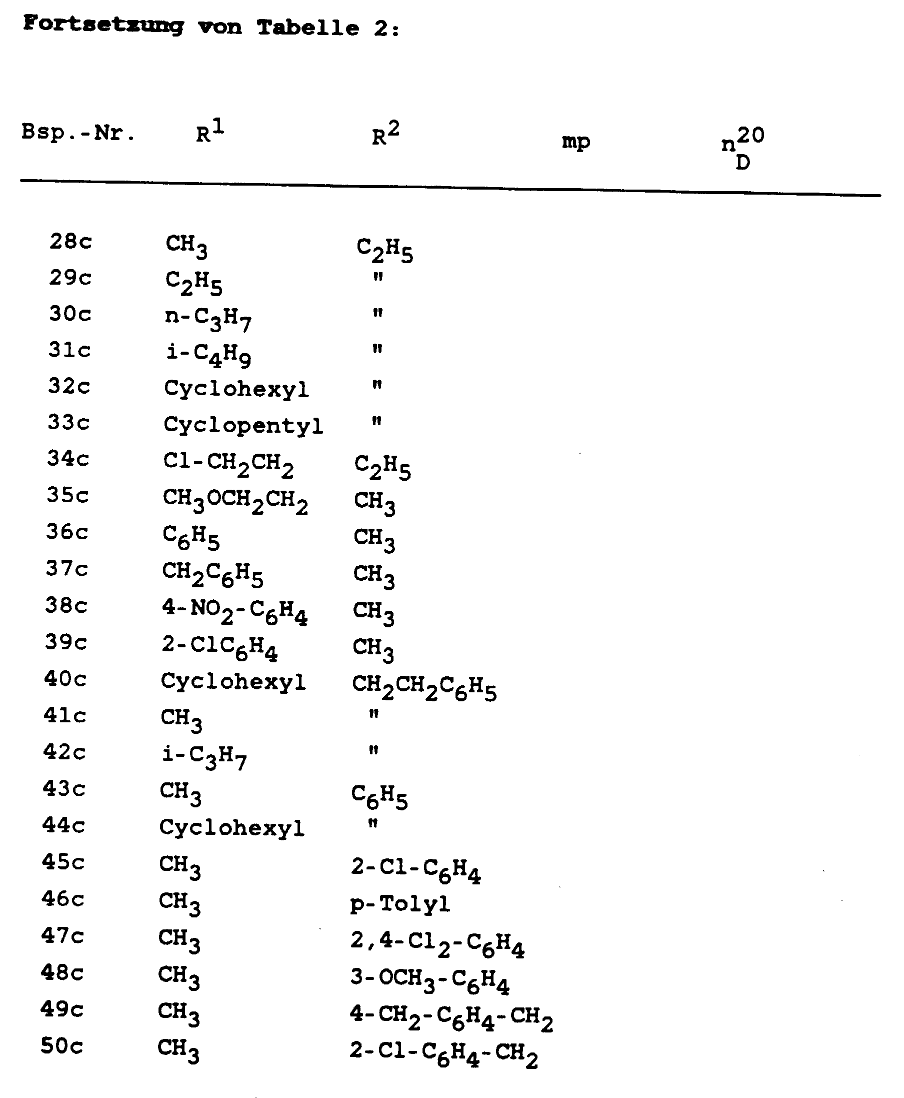

## Patentansprüche (Patentansprüche für folgende(n) Vertragsstaat(en): DE, GB, FR, IT, NL, SE, LI, CH, BE, AT, DK)

1. N-Acyl-2-aminosäuramide der allgemeinen Formel (I), 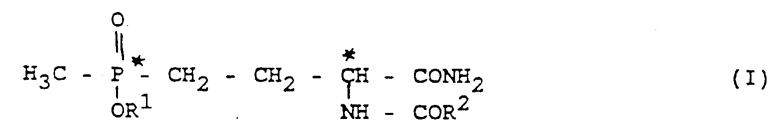 worin
R¹ C₁-C₁₄-Alkyl, welches verzweigt oder unverzweigt ist und unsubstituiert oder ein- oder mehrfach durch Halogen oder C₁-C₆-Alkoxy substituiert ist, Benzyl oder Phenyl, wobei Benzyl oder Phenyl im Phenylkern unsubstituiert oder ein- oder mehrfach durch C₁-C₄-Alkyl, C₁-C₄-Alkoxy, Halogen, Nitro oder Trifluormethyl substituiert sind, oder C₃-C₁₀-Cycloalkyl und
R² Wasserstoff, C₁-C₁₄-Alkyl, welches unsubstituiert ist oder ein- oder mehrfach durch Halogen oder C₁-C₄-Alkoxy substituiert ist, oder einen Rest der Formel -(CH₂)ₙ-Phenyl, der im Phenylkern unsubstituiert oder ein- bis dreifach durch C₁-C₄-Alkyl, C₁-C₄-Alkoxy, Halogen, Nitro oder Trifluormethyl substituiert ist und in dem n für 0, 1, 2 oder 3 steht,
bedeuten, ausgenommen die Verbindung der Formel (I), worin
R¹ = Ethyl und R² = Methyl oder
R¹ = Methyl und R² = Trifluormethyl bedeuten.

2. Verbindungen der Formel (I) nach Anspruch 1, dadurch gekennzeichnet, daß
R¹ unverzweigtes oder verzweigtes C₁-C₁₀-Alkyl oder C₁-C₁₀-Alkyl, das durch Halogen ein- bis dreifach substituiert ist, oder C₅-C₆-Cycloalkyl und
R² Wasserstoff, unverzweigtes oder verzweigtes C₁-C₁₄-Alkyl, das unsubstituiert oder durch ein bis drei Reste aus der Gruppe Halogen oder C₁-C₄-Alkoxy substituiert ist, oder ein Rest der Formel -(CH₂)ₙ-Phenyl, wobei der Phenylkern unsubstituiert oder ein- bis dreifach durch Halogen substituiert ist und n für 0, 1 oder 2 steht,
bedeuten.

3. Verbindungen der Formel (I) nach Anspruch 1 oder 2, dadurch gekennzeichnet, daß
R¹ unverzweigtes C₁-C₈-Alkyl oder Cyclohexyl und
R² C₁-C₄-Alkyl oder Benzyl
bedeuten.

4. Verfahren zur Herstellung der Verbindungen der Formel (I), 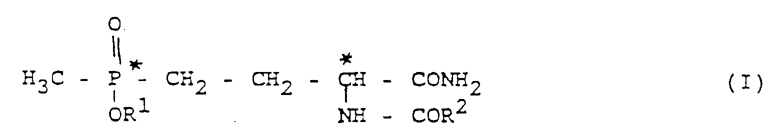 worin
R¹ C₁-C₁₄-Alkyl, welches verzweigt oder unverzweigt ist und unsubstituiert oder ein- oder mehrfach durch Halogen oder C₁-C₆-Alkoxy substituiert ist, Benzyl oder Phenyl, wobei Benzyl oder Phenyl im Phenylkern unsubstituiert oder ein- oder mehrfach durch C₁-C₄-Alkyl, C₁-C₄-Alkoxy, Halogen, Nitro oder Trifluormethyl substituiert sind, oder C₃-C₁₀-Cycloalkyl und
R² Wasserstoff, C₁-C₁₄-Alkyl, welches unsubstituiert ist oder ein- oder mehrfach durch Halogen oder C₁-C₄-Alkoxy substituiert ist, oder einen Rest der Formel -(CH₂)ₙ-Phenyl, der im Phenylkern unsubstituiert oder ein- bis dreifach durch C₁-C₄-Alkyl, C₁-C₄-Alkoxy, Halogen, Nitro oder Trifluormethyl substituiert ist und in dem n für 0, 1, 2 oder 3 steht,
bedeuten, dadurch gekennzeichnet, daß man N-Acyl-2-aminosäurenitrile der Formel (II), 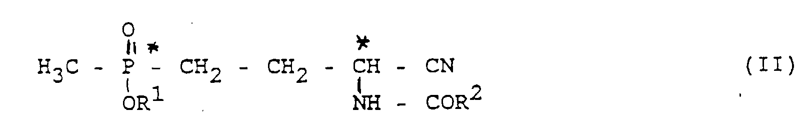 worin R¹ und R² die für Formel (I) angegebenen Bedeutungen haben, selektiv an der Nitril-Gruppe zum entsprechenden Carbonsäureamid der allgemeinen Formel (I) sauer hydrolysiert.

5. Verfahren nach Anspruch 4, dadurch gekennzeichnet, daß man die Hydrolyse in Ameisensäure oder wäßriger Ameisensäure durchführt.

6. Verfahren nach Anspruch 4, dadurch gekennzeichnet, daß man die Hydrolyse mit Ameisensäure in Gegenwart von Halogenwasserstoff durchführt.

7. Verfahren nach Anspruch 4, 5 oder 6, dadurch gekennzeichnet, daß man ein unter den Reaktionsbedingungen inertes Lösungsmittel verwendet.

8. Verfahren nach einem der Ansprüche 4 bis 7, dadurch gekennzeichnet, daß man die Hydrolyse bei 0 bis 250°C durchführt.

9. Verbindungen der Formel (II), 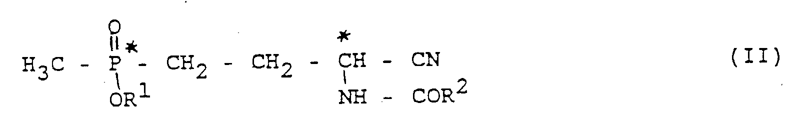 worin R¹ und R² die in Formel (I) nach Anspruch 4 definierten Bedeutungen haben.

10. Verfahren zur Herstellung der Verbindungen der Formel (II) nach Anspruch 9, dadurch gekennzeichnet, daß eine Verbindung der Formel (III) 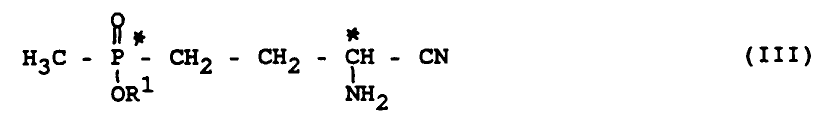 N-acyliert wird.

11. Verwendung von Verbindungen der Formel (I) nach einem oder mehreren der Ansprüche 1 bis 3 zur Herstellung von L- und D-Phosphinothricin und deren Derivaten sowie deren Gemischen, insbesondere von L-Phosphinothricin und -derivaten.

## Claims

1. An N-acyl-2-amino acid amide of the formula (I) 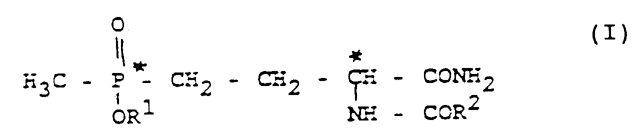 where
R¹ is C₁-C₁₄-alkyl which is branched or unbranched and unsubstituted or monosubstituted or polysubstituted by halogen or C₁-C₆-alkoxy, or is benzyl or phenyl, benzyl or phenyl being unsubstituted or monosubstituted or polysubstituted in the phenyl ring by C₁-C₄-alkyl, C₁-C₄-alkoxy, halogen, nitro or trifluoromethyl, or is C₃-C₁₀-cycloalkyl, and
R² is hydrogen, C₁-C₁₄-alkyl which is unsubstituted or monosubstituted or polysubstituted by halogen or C₁-C₄-alkoxy, or is a radical of the formula -(CH₂)ₙ-phenyl which is unsubstituted or monosubstituted to trisubstituted in the phenyl ring by C₁-C₄-alkyl, C₁-C₄-alkoxy, halogen, nitro or trifluoromethyl and in which n is 0, 1, 2 or 3,
with the exception of the compound of the formula (I) where
R¹ = ethyl and R² = methyl or
R¹ = methyl and R² = trifluoromethyl.

2. A compound of the formula (I) as claimed in claim 1, wherein
R¹ is unbranched or branched C₁-C₁₀-alkyl or C₁-C₁₀-alkyl which is monosubstituted to trisubstituted by halogen, or is C₅-C₆-cycloalkyl, and
R² is hydrogen, unbranched or branched C₁-C₁₄-alkyl which is unsubstituted or substituted by one to three radicals from the group comprising halogen and C₁-C₄-alkoxy, or is a radical of the formula -(CH₂)ₙ-phenyl, the phenyl ring being unsubstituted or monosubstituted to trisubstituted by halogen and n being 0, 1 or 2.

3. A compound of the formula (I) as claimed in claim 1 or 2, wherein
R¹ is unbranched C₁-C₈-alkyl or cyclohexyl, and
R² is C₁-C₄-alkyl or benzyl.

4. A process for the preparation of a compound of the formula (I) 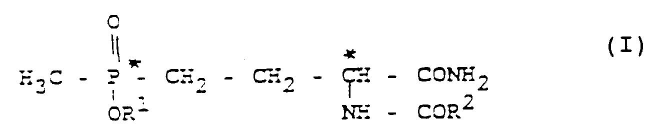 where
R¹ is C₁-C₁₄-alkyl which is branched or unbranched and unsubstituted or monosubstituted or polysubstituted by halogen or C₁-C₆-alkoxy, or is benzyl or phenyl, benzyl or phenyl being unsubstituted or monosubstituted or polysubstituted in the phenyl ring by C₁-C₄-alkyl, C₁-C₄-alkoxy, halogen, nitro or trifluoromethyl, or is C₃-C₁₀-cycloalkyl, and
R² is hydrogen, C₁-C₁₄-alkyl which is unsubstituted or monosubstituted or polysubstituted by halogen or C₁-C₄-alkoxy, or is a radical of the formula -(CH₂)ₙ-phenyl which is unsubstituted or monosubstituted to trisubstituted in the phenyl ring by C₁-C₄-alkyl, C₁-C₄-alkoxy, halogen, nitro or trifluoromethyl and in which n is 0, 1, 2 or 3,
which comprises subjecting an N-acyl-2-amino acid nitrile of the formula (II) 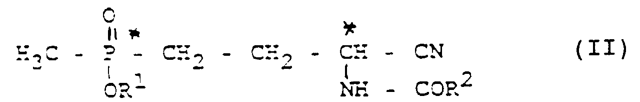 where R¹ and R² have the meanings indicated for formula (I), to selective acid hydrolysis on the nitrile group, to give the corresponding carboxamide of the formula (I).

5. The process as claimed in claim 4, wherein the hydrolysis is carried out in formic acid or aqueous formic acid.

6. The process as claimed in claim 4, wherein the hydrolysis is carried out using formic acid in the presence of hydrogen halide.

7. The process as claimed in claim 4, 5 or 6, wherein a solvent is used which is inert under the reaction conditions.

8. The process as claimed in any one of claims 4 to 7, wherein the hydrolysis is carried out at 0 to 250°C.

9. A compound of the formula (II) 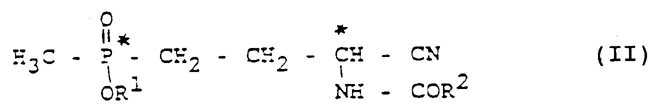 where R¹ and R² have the meanings defined in formula (I) as claimed in claim 4.

10. A process for the preparation of a compound of the formula (II) as claimed in claim 9, which comprises N-acylation of a compound of the formula (III)

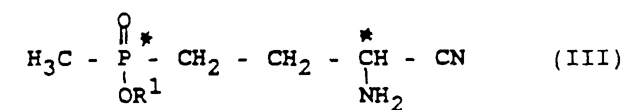

11. The use of a compound of the formula (I) as claimed in one or more of claims 1 to 3, for the preparation of Land D-phosphinothricin and a derivative thereof as well as a mixture thereof, in particular of L-phosphinothricin and an L-phosphinothricin derivative.

## Revendications

1. N-acyl-2-aminoamides de formule générale (I) 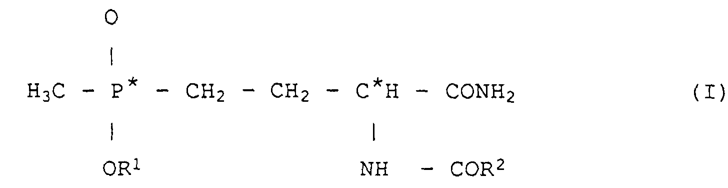 dans laquelle
R¹ est un groupe alkyle en C₁-C₁₄, à chaîne droite ou ramifiée, et qui est non substitué ou une ou plusieurs fois substitué par des substituants halogéno ou alcoxy en C₁-C₆, benzyle ou phényle, les groupes benzyle ou phényle étant non substitués sur le noyau phényle, ou étant une ou plusieurs fois substitués par des substituants alkyle en C₁-C₄, alcoxy en C₁-C₄, halogéno, nitro ou trifluorométhyle, ou encore cycloalkyle en C₃-C₁₀, et
R² est un hydrogène, un groupe alkyle en C₁-C₁₄ qui est non substitué ou qui est une ou plusieurs fois substitué par des substituants halogéno ou alcoxy en C₁-C₄, ou encore, un radical de formule -(CH₂)ₙ-phényle, qui est non substitué dans le noyau phényle ou est une à trois fois substitué par des substituants alkyle en C₁-C₄, alcoxy en C₁-C₄, halogéno, nitro ou trifluorométhyle, et où n vaut 0, 1, 2 ou 3,
à l'exclusion du composé de formule (I) dans laquelle R¹ est le radical éthyle et R² est le radical méthyle, ou R¹ est le radical méthyle et R² est le radical trifluorométhyle.

2. Composés de formule (I) selon la revendication 1, caractérisés en ce que
R¹ est un groupe alkyle en C₁-C₁₀ à chaîne droite ou ramifiée, ou un groupe alkyle en C₁-C₁₀ une à trois fois substitué par des substituants halogéno, ou bien un groupe cycloalkyle en C₅-C₆, et
R² est un hydrogène, un groupe alkyle en C₁-C₁₄ à chaîne droite ou ramifiée qui est non substitué ou est une à trois fois substitué par des radicaux choisis parmi les groupes halogéno ou alcoxy en C₁-C₄, ou encore un radical de formule -(CH₂)ₙ-phényle, où le noyau phényle est non substitué, ou est une à trois fois substitué par des halogènes, et n vaut 0, 1 ou 2.

3. Composés de formule (I) selon la revendication 1 ou 2, caractérisés en ce que
R¹ est un groupe alkyle en C₁-C₈ à chaîne droite ou cyclohexyle, et
R² est un groupe alkyle en C₁-C₄ ou le groupe benzyle.

4. Procédé pour préparer les composés de formule (I) 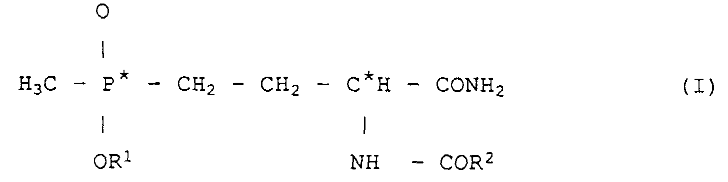 dans laquelle
R¹ est un groupe alkyle en C₁-C₁₄, à chaîne droite ou ramifiée, et qui est non substitué ou une ou plusieurs fois substitué par des substituants halogéno ou alcoxy en C₁-C₆, benzyle ou phényle, les groupes benzyle ou phényle étant non substitués sur le noyau phényle, ou étant une ou plusieurs fois substitués par des substituants alkyle en C₁-C₄, alcoxy en C₁-C₄, halogéno, nitro ou trifluorométhyle, ou encore cycloalkyle en C₃-C₁₀, et
R² est un hydrogène, un groupe alkyle en C₁-C₁₄ qui est non substitué ou qui est une ou plusieurs fois substitué par des substituants halogéno ou alcoxy en C₁-C₄, ou encore, un radical de formule -(CH₂)ₙ-phényle, qui est non substitué dans le noyau phényle ou est une à trois fois substitué par des substituants alkyle en C₁-C₄, alcoxy en C₁-C₄, halogéno, nitro ou trifluorométhyle, et où n vaut 0, 1, 2 ou 3, caractérisé en ce qu'on hydrolyse en milieu acide des N-acyl-2-aminonitriles de formule (II) 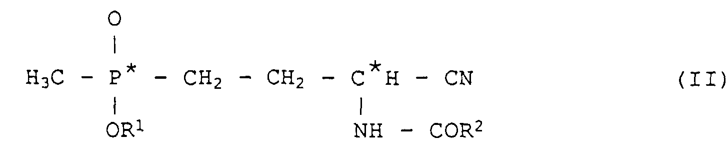
dans laquelle R¹ et R² ont les significations données pour la formule (I), d'une manière sélective sur le groupe nitrile, pour obtenir le carboxamide correspondant de formule générale (I).

5. Procédé selon la revendication 4, caractérisé en ce que l'hydrolyse est mise en oeuvre dans de l'acide formique ou dans de l'acide formique en aqueux.

6. Procédé selon la revendication 4, caractérisé en ce que l'hydrolyse avec l'acide formique est mise en oeuvre en présence d'un acide halohydrique.

7. Procédé selon la revendication 4, 5 ou 6, caractérisé en ce qu'on utilise un solvant inerte dans les conditions de la réaction.

8. Procédé selon l'une des revendications 4 à 7, caractérisé en ce qu'on met en oeuvre l'hydrolyse à une température de 0 à 250°C.

9. Composés de formule (II) 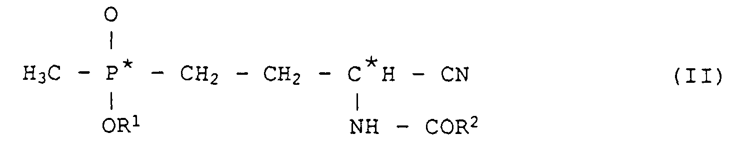 dans laquelle R¹ et R² ont les significations données pour la formule (I) selon la revendication 4.

10. Procédé pour préparer les composés de formule (II) selon la revendication 9, caractérisé en ce qu'on soumet à une N-acylation un composé de formule (III)

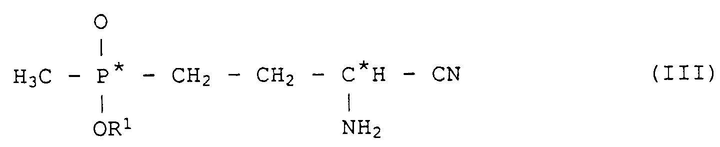

11. Utilisation de composés de formule (I) selon l'une ou plusieurs des revendications 1 à 3 pour préparer la L- et la D-phosphinothricine et leurs dérivés et leurs mélanges, en particulier la L-phosphinothricine et ses dérivés.
